# EUROPEAN PATENT APPLICATION

(11) **EP 3 556 932 A1**
(43) Date of publication of application: **23.10.2019**
(21) Application number: 19176121.2
(22) Date of filing: 01.04.2015
(51) Int. Cl.: D06M 15/53, A61F 13/53, D06M 13/02, D06M 13/165, D06M 13/224, D06M 101/08

(54) **HYDROPHILIZED CELLULOSE ACETATE TOW BAND, AND ABSORBENT MATERIAL PRODUCED USING SAME**

(30) Priority: 01.04.2014 JP 2014075094
(62) Divisional of application: 15773559.8
(71) Applicant: Daicel Corporation, Osaka 530-0011 (JP)
(72) Inventor: NAKAMURA, Toshikazu, Himeji-shi, Hyogo 671-1283 (JP); SHIGEMATSU, Masato, Himeji-shi, Hyogo 671-1283 (JP)
(74) Representative: Grünecker Patent- und Rechtsanwälte PartG mbB

(57) **Abstract**

A hydrophilized cellulose acetate tow band according to the present invention includes a crimped cellulose acetate tow band, and a hydrophilizing component having an HLB value of 16 or more. The cellulose acetate tow band includes a cellulose acetate having a degree of substitution of 2.0 to 2.6. The cellulose acetate tow band has a total denier of 10000 to 40000 and is crimped in a number of crimps of 30 to 60 per inch. The hydrophilizing component is impregnated on the cellulose acetate tow band in an amount of 0.3 to 2 weight percent relative to the cellulose acetate tow band. The hydrophilized cellulose acetate tow band may further include a textile oil, where the textile oil is impregnated on the crimped cellulose acetate tow band in an amount of 0.2 to 2 weight percent relative to the cellulose acetate tow band, in addition to the hydrophilizing component having an HLB value of 16 or more.

## Description

### Technical Field

The present invention relates to a hydrophilized cellulose acetate tow band which is useful as absorbing materials for absorbents for use in hygiene supplies such as diapers and feminine sanitary products. The present invention also relates to a fiber sheet derived from (formed from) the hydrophilized cellulose acetate tow band; and to an absorbent (absorbent material) including the fiber sheet. This application claims priority to Japanese Patent Application No. 2014-75094 filed to Japan April 1, 2014, the entire contents of which are incorporated herein by reference.

### Background Art

Conventional absorbents for use in sanitary products such as disposable diapers include fluff pulp and a polymer absorbent such as a superabsorbent polymer (SAP). Such absorbents have large thicknesses and are uneasy to carry. In addition, the fluff pulp absorbs water by its constitutive fibers themselves, and this disadvantageously causes a low liquid permeability rate.

As a possible solution to the problem of the conventional absorbents using fluff pulp, absorbents mainly containing a cellulose acetate in combination with a SAP are proposed. For example, Patent Literature (PTL) 1 discloses a method for producing an absorbing composite. The method includes spreading a crimped tow in a direction perpendicular to the direction of tow travel. The spread tow is deregistered and shaped so as to have a substantially rectangular cross-section. Particles typically of a superabsorbent polymer are entirely distributed within the shaped tow. A non-limiting example of the tow is a cellulose acetate tow produced for cigarette filters. Such an absorbent using a cellulose acetate can be reduced in thickness and give disposable diapers which are convenient to carry. In addition, the absorbent has excellent permeability and utilizes the SAP effectively. Disadvantageously, however, such a thin absorbent using a cellulose acetate has insufficient liquid-absorption properties, in particular insufficient liquid diffusibility and liquid retentivity. Specifically, the absorbent is disadvantageous in that a liquid such as urine is absorbed not by a wide-spreading area, but by a concentrated local portion; and that when the absorbent is inclined, the liquid is liable to leak because of low liquid retentivity. In other words, the absorbent has somewhat inferior liquid diffusibility and liquid retentivity among the liquid absorbing functions including liquid permeation, liquid diffusion, and liquid retention. Thus, the absorbent has poor balance among the liquid absorbing functions.

PTL 2 discloses an absorbent article as a technique relating to absorbents using filaments other than cellulose acetate filaments. The absorbent article includes a nonwoven fabric as a material to be in contact with the skin. The nonwoven fabric mainly includes polyolefin or polyester fibers each having a sericin-treated surface. PTL 3 discloses a wet laid web of cellulose fibers for use in production of an air laid mass of fibers. The wet laid web of cellulose fibers includes cellulose fibers; and a fiber treatment composition including a polysaccharide and at least one agent having hydrogen bonding functionality. The fiber treatment composition is distributed within the wet laid web of cellulose fibers. PTL 4 discloses a bundle of synthetic fibers for transporting aqueous fluids. The fibers are coated with a lubricant including 10 weight percent of a solution of poly[polyethylene-glycol (1400) terephthalate], 44.1 weight percent of polyethylene glycol (400) monolaurate, 44.1 weight percent of polyethylene glycol (600) monolaurate, and 1.8 weight percent of 4-cetyl, 4-ethyl morpholinium ethosulfate.

PTL 5 discloses an absorbent article including fibers having parameters meeting specific conditions. This literature describes, as a particular preferred hydrophilic lubricant usable for lubricating the fibers, a lubricant including 49% of poly(ethylene glycol) (PEG) 600 monolaurate or polyoxyethylene (13.64) monolaurate, 49% of poly(ethylene glycol) (PEG) 400 monolaurate or polyoxyethylene (9.09) monolaurate, and 2% of 4-cetyl-4-ethylmorpholinium ethosulfate. This literature gives polyesters as preferred materials for use in production of the fibers and lists polyamides, polypropylenes, polyethylenes, and cellulose esters as other materials for the production.

In general, a textile oil is imparted to traveling filaments in a fiber production process (spinning process; fiber forming process), in the field of cellulose acetates for cigarette filters (cigarette filter tows) as described in PTL 6. This is performed so that the filaments are bundled more satisfactorily and have better properties such as smoothness and antistaticity. The textile oil includes an oil component as a principal component, in combination with one or more additives such as surfactants as needed. Such techniques for imparting a textile oil in the spinning process place importance on uniform coating of the textile oil. This is because the textile oil uniform coating allows the filaments to be bundled more satisfactorily and to have better properties such as smoothness and antistaticity; and eliminates or minimizes following disadvantages caused by abrasion (wear). Specifically, contact wear with equipment damages fibers and causes the fibers to have lower quality. In addition, fibers are cut into fiber pieces by abrasion, and the fiber pieces adversely affect the working environment and lower the quality of products. In the cigarette filter tows, the tows have to be crimped so as to offer a pressure drop at certain level, and the tows essentially contain moisture in a certain amount so as to be crimped. Accordingly, a textile oil emulsion is used in the spinning (fiber forming) of filter tows, where the textile oil emulsion is a suspension prepared by diluting the textile oil with water, namely, a suspension of water and oil. The textile oil emulsion contains the textile oil in a concentration of about 2 to about 10 weight percent.

### Citation List

### Patent Literature

PTL 1: Japanese Unexamined Patent Application Publication (JP-A) No. 2004-244794
PTL 2: JP-A No. H09-322911
PTL 3: JP-A No. 2004-143653
PTL 4: Japanese Unexamined Patent Application Publication (Translation of PCT Application) (JP-A) No. 2001-500198
PTL 5: JP-A No. 2003-33398
PTL 6: JP-A No. 2007-77525

### Summary of Invention

### Technical Problem

The present invention has an object to provide a cellulose acetate absorbing material that can be reduced in thickness and has liquid permeability, liquid diffusibility, and liquid retentivity all at high levels in good balance. The present invention has another object to provide a fiber sheet made from the cellulose acetate absorbing material, and an absorbent including the fiber sheet.

### Solution to Problem

After intensive investigations to achieve the objects, the inventors of the present invention found a cellulose acetate tow band that includes a crimped cellulose acetate tow band and a hydrophilizing component having an HLB value at a specific level or more and being impregnated onto the crimped cellulose acetate tow band, where the cellulose acetate tow band includes (made of) a cellulose acetate having a specific degree of substitution and has a total denier within a specific range. The inventors found that this is usable as an absorbing material that can be reduced in thickness and has liquid permeability, liquid diffusibility, and liquid retentivity all at high levels in good balance. The present invention has been made based on these findings.

Specifically, the present invention provides, in an embodiment, a hydrophilized cellulose acetate tow band including a crimped cellulose acetate tow band, and a hydrophilizing component. The cellulose acetate tow band includes a cellulose acetate having a degree of substitution of 2.0 to 2.6, has a total denier of 10000 to 40000, and is crimped in a number of crimps of 30 to 60 per inch. The hydrophilizing component has an HLB value of 16 or more and is impregnated on the cellulose acetate tow band in an amount of 0.2 to 2 weight percent relative to the cellulose acetate tow band.

In the hydrophilized cellulose acetate tow band, the hydrophilizing component having an HLB value of 16 or more may be at least one selected from the group consisting of polyoxyethylene sorbitan monolaurates and polyethylene glycol monostearates.

The hydrophilized cellulose acetate tow band may further include a textile oil. The textile oil is impregnated on the crimped cellulose acetate tow band in an amount of 0.2 to 2 weight percent relative to the cellulose acetate tow band, in addition to the hydrophilizing component having an HLB value of 16 or more.

In the hydrophilized cellulose acetate tow band, the hydrophilizing component having an HLB value of 16 or more and the textile oil may be impregnated in a total amount of 0.4 to 3 weight percent relative to the cellulose acetate tow band.

In the hydrophilized cellulose acetate tow band, the crimped cellulose acetate tow band may have a filament denier of 1.0 to 7.0.

The present invention provides, in another embodiment, a fiber sheet derived from the hydrophilized cellulose acetate tow band.

The present invention provides, in yet another embodiment, an absorbent including the fiber sheet and a superabsorbent polymer. The superabsorbent polymer is dispersively disposed within and over the fiber sheet.

The present invention provides, in still another embodiment, a method for producing a hydrophilized cellulose acetate tow band. The method includes the steps (A), (B), (C), (D), and (E). In the step (A), a cellulose acetate having a degree of substitution of 2.0 to 2.6 is dissolved in an organic solvent to give a spinning dope, and the spinning dope is discharged through spinnerets of spinning cabinets to give cellulose acetate filaments. In the step (B), the cellulose acetate filaments are coated with a textile oil. In the step (C), the cellulose acetate filaments are coated with a hydrophilizing component. In the step (D), the cellulose acetate filaments from the spinning cabinets are unified to give a cellulose acetate tow band having a total denier of 10000 to 40000. In the step (E), the cellulose acetate tow band is crimped to give a crimped cellulose acetate tow band being crimped in a number of crimps of 30 to 60 per inch.

The method for producing a hydrophilized cellulose acetate tow band may be performed so that the cellulose acetate filaments is coated with both the textile oil and the hydrophilizing component in the step (B); and that the cellulose acetate filaments coated with the textile oil and the hydrophilizing component from the step (B) are further coated with the hydrophilizing component in the step (C).

In the method for producing a hydrophilized cellulose acetate tow band, the step (B), the step (D), and the step (C) may be performed in this sequence.

### Advantageous Effects of Invention

The hydrophilized cellulose acetate tow band according to the present invention includes a crimped cellulose acetate tow band, and a hydrophilizing component having an HLB value of 16 or more, where the hydrophilizing component is impregnated in the specific amount on the crimped cellulose acetate tow band. The crimped cellulose acetate includes (made of) a cellulose acetate having a degree of substitution of 2.0 to 2.6, has a total denier of 10000 to 40000, and is crimped in a number of crimps of 30 to 60 per inch. This configuration allows the hydrophilized cellulose acetate tow band to maintain liquid permeability, which is a feature of such cellulose acetate tows, and to still have better liquid absorbing properties, in particular, better liquid diffusibility and liquid retentivity (wetting properties), all in good balance. The hydrophilized cellulose acetate tow band, as employing a cellulose acetate tow, is not bulky and can be reduced in thickness. The hydrophilized cellulose acetate tow band is therefore usable as absorbing materials for absorbents for use in hygiene supplies such as diapers and feminine sanitary products.

The cellulose acetate having a degree of substitution of 2.0 to 2.6 is not soluble in water, and, accordingly, the surface of the cellulose acetate is not soluble in water. However, the hydrophilizing component having an HLB value of 16 or more is soluble in water. The hydrophilizing component having an HLB value of 16 or more, when coated onto the cellulose acetate, allows the surface of the cellulose acetate to be hydrophile.

The absorbent according to the present invention includes a fiber sheet, and a superabsorbent polymer (SAP) dispersively disposed within and over the fiber sheet, where the fiber sheet is made from (includes) the hydrophilized cellulose acetate tow. The absorbent maintains liquid permeability, which the cellulose acetate tow inherently has, absorbs a liquid in a short time, and can absorb, for example, voided urine in a short time. The absorbent has excellent liquid diffusibility, utilizes the SAP in a wide area, thereby less suffers from rewetting typically of urine, and is comfortable to use. In addition, the absorbent has excellent liquid retentivity and restrains spillage of the liquid sideways due typically to baby's motion.

The method according to the present invention for producing a hydrophilized cellulose acetate tow industrially efficiently produces a cellulose acetate tow that has liquid permeability and liquid absorbing properties both at excellent levels and is hydrophilized at its surface.

### Brief Description of Drawings

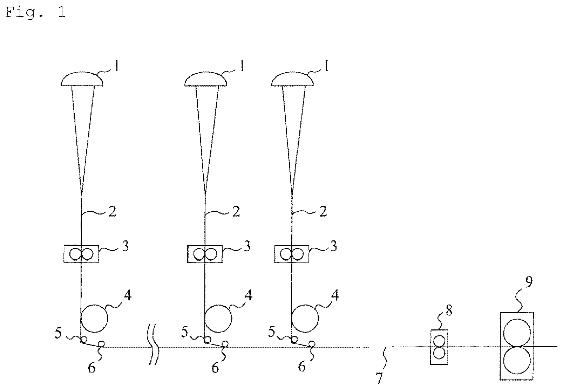

Fig. 1 schematically illustrates a representation of equipment for use in the method according to the present invention for producing a hydrophilized cellulose acetate tow band.

### Description of Embodiments

### Hydrophilized Cellulose Acetate Tow Band

The hydrophilized cellulose acetate tow band according to the present invention includes a crimped cellulose acetate tow band, and a hydrophilizing component. The crimped cellulose acetate tow band includes (made of) a cellulose acetate having a degree of substitution of 2.0 to 2.6, has a total denier of 10000 to 40000, and is crimped in a number of crimps of 30 to 60 per inch. The hydrophilizing component has an HLB value of 16 or more and is impregnated on the crimped cellulose acetate tow band. The cellulose acetate tow band in the present invention has only to be crimped and impregnated with the hydrophilizing component having an HLB value of 16 or more in the final form. Thus, the operations of crimping and hydrophilizing component impregnating may be performed in any order not limited.

The cellulose acetate constituting the cellulose acetate tow band in the present invention has a degree of substitution (degree of acetyl substitution) of 2.0 to 2.6, and preferably 2.1 to 2.5.

The cellulose acetate fibers constituting the cellulose acetate tow band are generally continuous fibers (filaments) produced by dry spinning. The cross-sectional shape and fineness of the fibers may vary depending on the spinneret shape and other spinning conditions.

The cellulose acetate fibers may have a round cross section or a cross section of any odd (not-round) shape, such as elliptic, polygonal, T-shaped, I-shaped, Y-shaped, or X-shaped cross section. Fibers having such an odd-shaped cross section have larger liquid absorbing ability and is more suitable as an absorbing material, as compared with fibers having a round cross section. In particular, the cellulose acetate fibers preferably have Y-shaped cross sections.

The cellulose acetate fibers may have any monofilament fineness (single fiber fineness) not limited, but typically 0.1 to 20 deniers, and preferably 1.0 to 7.0 deniers.

As described above, the cellulose acetate tow band in the present invention is a bundle of crimped continuous fibers (filaments). The cellulose acetate tow band has a total denier of 10000 to 40000, preferably 15000 to 35000, and more preferably 20000 to 30000. The cellulose acetate tow band, if having a total denier less than 10000, may be hardly crimped uniformly and, when processed into an absorbent, may hardly maintain its shape as the absorbent. In contrast, the cellulose acetate tow band, if having a total denier greater than 40000, may disadvantageously give a large thickness and offer a stiff or rough feeling when processed into an absorbent.

The crimped cellulose acetate tow band has a number of crimps of 30 to 60 per inch, and preferably 30 to 40 per inch. The cellulose acetate tow band, if having a number of crimps less than 30 per inch, may expand insufficiently when spread. In contrast, the cellulose acetate tow band, if having a number of crimps greater than 60 per inch, may disadvantageously be damaged due to excessively high crimping.

The impregnating of the hydrophilizing component having an HLB value of 16 or more onto the cellulose acetate tow band is important in the present invention, as described above.

The hydrophilizing component having an HLB value of 16 or more is not limited, but is preferably selected from nonionic surfactants having an HLB value of 16 or more. More specifically, non-limiting examples of the hydrophilizing component having an HLB value of 16 or more include polyoxyethylene sorbitan monolaurates (e.g., Tween 20) (HLB value: 16.7) and polyethylene glycol monostearates (HLB value: 18.0). The crimped cellulose acetate tow band may include each of different hydrophilizing components having an HLB value of 16 or more alone or in combination.

The hydrophilizing component preferably has an HLB value of 16.5 or more. The upper limited of the HLB value of the hydrophilizing component is not limited, but is generally 20.0, and may be 19.0.

The hydrophilizing component having an HLB value of 16 or more is preferably selected from the polyoxyethylene sorbitan monolaurates (HLB value: 16.7) because of having broad utility.

The hydrophilizing component having an HLB value of 16 or more is impregnated on the cellulose acetate tow band in an amount of 0.2 to 2 weight percent, and preferably 0.25 to 1 weight percent, relative to the cellulose acetate tow band. As the hydrophilizing component having an HLB value of 16 or more is impregnated on the cellulose acetate tow band in an amount within the range, the hydrophilized cellulose acetate tow band maintains liquid permeability, which the cellulose acetate tow band inherently has, and still has liquid diffusibility and liquid retentivity (wetting properties) at significantly higher levels.

A way to treat the cellulose acetate tow band surface with the hydrophilizing component having an HLB value of 16 or more is not limited. For example, the hydrophilizing component having an HLB value of 16 or more can be imparted to (impregnated on) the cellulose acetate tow band surface by a common surface treatment technique such as immersion, spraying, or coating technique, using an aqueous solution of the hydrophilizing component having an HLB value of 16 or more in water.

The cellulose acetate tow band in the present invention has only to be impregnated, on its surface, with the hydrophilizing component having an HLB value of 16 or more in the specific amount. However, the cellulose acetate tow band surface is preferably further impregnated with a textile oil. This is preferred because the resulting cellulose acetate tow band has better spinnability, its constitutive filaments can be bundled more satisfactorily, and the fiber surface has smoothness, antistaticity, and antifriction properties at higher levels.

The textile oil is not limited and may be selected from known or common textile oils. Non-limiting examples of the textile oil include mineral oils and esterified oils. Among them, mineral oils are preferred. The crimped cellulose acetate tow band may include each of different textile oils alone or in combination.

The amount of the textile oil to be impregnated on the cellulose acetate tow band may be selected depending typically on the types of the cellulose acetate tow band and the textile oil, and the treatment technique as appropriate. In general, the amount is typically 0.2 to 2 weight percent, and preferably 0.25 to 1 weight percent, relative to the cellulose acetate tow band.

A way to treat the cellulose acetate tow band surface with the textile oil is not limited. For example, the textile oil can be imparted to (impregnated on) the cellulose acetate tow band surface by a common surface treatment technique such as immersion, spraying, or coating technique, using the textile oil as intact, or a solution of the textile oil dissolved in an organic solvent, or an emulsion of the textile oil suspended in water.

The emulsion of the textile oil suspended in water (textile oil emulsion), when used, may be combined with a surfactant as needed. Non-limiting examples of the surfactant include anionic surfactants; and nonionic surfactants having an HLB value less than 16.

The textile oil emulsion is preferably a textile oil emulsion that has a total luminous transmittance of 30% or more to light at 850 nm, where the total luminous transmittance is measured at an emulsion concentration of 5 weight percent (see JP-A No. 2007-77525). The impregnating of the cellulose acetate filament tows with the textile oil emulsion as mentioned above significantly restrains deterioration in quality due to fiber damage by contact wear with equipment during the fiber production (formation) process. The impregnating also significantly restrains deterioration of the working environment due to fiber pieces cut as a result of wear. In addition, the impregnating reduces the friction force between spinning guides and the tow band upon spinning filaments and significantly reduces the frequency of end breakage on godet rollers in the spinning process.

The hydrophilizing component having an HLB value of 16 or more and the textile oil, when both impregnated on the cellulose acetate tow band surface, may be impregnated in a total amount of typically 0.4 to 3 weight percent, preferably 0.5 to 2 weight percent, more preferably 0.5 to 1.5 weight percent, and furthermore preferably 0.6 to 1.2 weight percent. The hydrophilizing component having an HLB value of 16 or more and the textile oil, if impregnated in an excessively large total amount, may cause troubles such as loose yarn and spinning dope dropping in the production process.

The hydrophilized cellulose acetate tow band according to the present invention includes the crimped cellulose acetate tow band, and the hydrophilizing component having an HLB value of 16 or more impregnated on the crimped cellulose acetate tow band, where the crimped cellulose acetate tow band has a total denier within the specific range and a number of crimps within the specific range. The hydrophilized cellulose acetate tow band therefore maintains liquid permeability, which is an inherent property of the cellulose acetate tow, and still has liquid absorbing properties, in particular liquid diffusibility and liquid retentivity (wetting properties) at higher levels in good balance. The hydrophilized cellulose acetate tow band, as using the cellulose acetate tow band, is not bulky and can be reduced in thickness. This allows the hydrophilized cellulose acetate tow band to be usable as absorbing materials for absorbents for use in hygiene supplies such as diapers and feminine sanitary products.

The hydrophilized cellulose acetate tow band according to the present invention may be produced typically by a method mentioned below.

### Production of Hydrophilized Cellulose Acetate Tow Band

The hydrophilized cellulose acetate tow band can be produced by treating the cellulose acetate tow band surface with a hydrophilizing component (in particular, a hydrophilizing component having an HLB value of 16 or more). The cellulose acetate tow band surface is preferably further treated with a textile oil, in addition to the hydrophilizing component. This is preferred because the resulting cellulose acetate tow band offers better spinnability, its constitute filaments can be bundled more satisfactorily, and the fiber surface can have smoothness, antistaticity, and antifriction properties at higher levels.

A non-limiting example of the method for producing such a hydrophilized cellulose acetate tow band including a cellulose acetate tow band; and a hydrophilizing component and a textile oil both imparted to (impregnated on) the surface of the cellulose acetate tow band is a method that produces a hydrophilized cellulose acetate tow band via the steps (A), (B), (C), (D), and (E) as follows.

In the step (A), a cellulose acetate having a degree of substitution of 2.0 to 2.6 is dissolved in an organic solvent to give a spinning dope, and the spinning dope is discharged through spinnerets of spinning cabinets to form cellulose acetate filaments.

In the step (B), the cellulose acetate filaments are coated with a textile oil.

In the step (C), the cellulose acetate filaments are coated with a hydrophilizing component.

In the step (D), the cellulose acetate filaments from the spinning cabinets are unified to give a cellulose acetate tow band having a total denier of 10000 to 40000.

In the step (E), the cellulose acetate tow band is crimped to give a crimped cellulose acetate tow band having a number of crimps of 30 to 60 per inch.

In the production method, the textile oil coating (imparting) (the step (B)) and the hydrophilizing component coating (imparting) (the step (C)) may be performed concurrently, or successively (one after another), or in combination. In the successive coating, the two components may be coated in any order, but preferably the textile oil is coated in first. The step (D) of forming a tow band may be performed either upstream or downstream from the step (B) and either upstream or downstream from the step (C). The step (B), the step (D), and the step (C) herein are preferably performed in this sequence.

Exemplary embodiments of the production method include methods I, II, and III as follows.

### Method I

In the method I, the cellulose acetate filaments from the step (A) are coated with the textile oil (without coating with the hydrophilizing component) in the step (B); and the cellulose acetate filaments coated with the textile oil are coated with the hydrophilizing component in the step (C) .

### Method II

In the method II, the cellulose acetate filaments from the step (A) are coated with both the textile oil and the hydrophilizing component in the step (B). In other words, the step (B) and the step (C) are performed simultaneously in the method II.

### Method III

In the method III, the cellulose acetate filaments from the step (A) are coated with both the textile oil and the hydrophilizing component in the step (B); and the cellulose acetate filaments coated with both the textile oil and the hydrophilizing component are further coated with the hydrophilizing component in the step (C).

Among the methods, the method III is particularly preferred. This is because the method III allows the hydrophilizing component to be present at the outermost surface to offer liquid absorbing properties, in particular, liquid diffusibility and liquid retentivity (wetting properties), at significantly better levels. In addition, a mixture of the textile oil and the hydrophilizing component is coated, and thereafter the hydrophilizing component is further coated according to the method III. This configuration enables more easy coating of a portion of the hydrophilizing component to be coated later. In the method III, the amount of the hydrophilizing component for use in the step (C) is typically 5 to 95 weight percent, preferably 30 to 90 weight percent, and more preferably 50 to 85 weight percent, based on the total amount of the hydrophilizing component (total amount to be used in the steps (B) and (C)).

In all of the methods, the step (B) is preferably performed so that the textile oil (or a mixture of the textile oil and the hydrophilizing component) is impregnated onto a cellulose acetate filament tow which is a ropelike bundle of a multiplicity of cellulose acetate filaments.

The step (C) is preferably performed so that the hydrophilizing component is impregnated onto a tow band which is formed by unifying cellulose acetate filaments (in particular, cellulose acetate filament tows) from two or more spinning cabinets. Namely, the hydrophilizing component impregnating in the step (C) is preferably performed after the step (D). This is preferred typically from the viewpoint of production efficiency.

Fig. 1 schematically illustrates a representation of equipment for use in the method according to the present invention for producing a hydrophilized cellulose acetate tow band.

The equipment illustrated in Fig. 1 employs two or more spinning cabinets, in each of which a multiplicity of cellulose acetate filaments are spun from spinnerets 1 and are bundled to give a ropelike cellulose acetate filament tow 2. The cellulose acetate filament tows 2 from the spinning cabinets are bundled into a band to give a cellulose acetate tow band 7.

The equipment includes textile-oil-imparting devices 3, a tow-band-forming means (tow-band-forming device) 6, a hydrophilizing-component-imparting device 8, and a crimper 9. The textile-oil-imparting devices 3 each impart the cellulose acetate filament tow 2 with a textile oil (or a mixture of the textile oil and the hydrophilizing component). The tow-band-forming device 6 bundles the cellulose acetate filament tows 2, which are obtained from the spinning cabinets and imparted with the textile oil (or a mixture of the textile oil and the hydrophilizing component), into a band to give the cellulose acetate tow band 7. The hydrophilizing-component-imparting device 8 imparts the hydrophilizing component (in particular, a hydrophilizing component having an HLB value of 16 or more) to the cellulose acetate tow band 7 formed by the tow-band-forming device 6. The crimper 9 crimps (imparts crimping to) the cellulose acetate tow band 7 imparted with the hydrophilizing component. The equipment further includes godet rollers 4 and guides 5 so as to smoothly form the cellulose acetate tow band 7 from the cellulose acetate filament tows 2 imparted with the textile oil. The hydrophilizing-component-imparting device 8 is not necessary in the method II.

The textile-oil-imparting devices 3 are not limited, as long as being capable of imparting the textile oil (or a mixture of the textile oil and the hydrophilizing component) to the cellulose acetate filament tow 2, and may be selected from known or common textile-oil-imparting devices (textile oil impregnating devices). Non-limiting examples of the textile-oil-imparting devices 3 include imparting devices of roller, nozzle, slit, and any other systems.

The hydrophilizing-component-imparting device 8 is not limited, as long as being capable of imparting the hydrophilizing component (in particular, the hydrophilizing component having an HLB value of 16 or more) to the cellulose acetate filament tow 2. For example, the textile-oil-imparting device (textile oil impregnating device) may be diverted. Specifically, the textile-oil-imparting device (textile oil impregnating device) is usable as the hydrophilizing-component-imparting devices 8 by using the hydrophilizing component instead of the textile oil (or a mixture of the textile oil and the hydrophilizing component) .

In this embodiment, the imparting (impregnating) of hydrophilizing component to (onto) the cellulose acetate tow band surface is performed at a stage after the textile oil imparting (impregnating) step and the tow band forming step and before the crimping step. The imparting (impregnating) may be performed at any appropriate step (stage), not limited to the aforementioned stage. However, the imparting (impregnating) of the hydrophilizing component to the cellulose acetate tow is preferably performed after the imparting (impregnating) of the textile oil (in particular, after forming the cellulose acetate tows imparted with the textile oil into a tow band). This is because the spinnability, filament bundling properties, fibers surface smoothness, antistaticity, and antifriction properties are desirably imparted to the cellulose acetate tows at early stages. The imparting (impregnating) of the hydrophilizing component onto the cellulose acetate tow band is preferably performed before the crimping step. This is preferred for uniform impregnating of the hydrophilizing component onto the cellulose acetate tow band.

The crimper 9 is not limited and may be selected from known or common crimpers. The crimper 9 gives sufficient crimping (crimper treatment) to the cellulose acetate tow band 7.

### Fiber Sheet

The fiber sheet according to the present invention is made from (derived from) the hydrophilized cellulose acetate tow band according to the present invention.

The fiber sheet may be produced typically by spreading the crimped cellulose acetate tow band (cellulose acetate tow band after crimping) and shaping the spread article into a sheet.

The crimped cellulose acetate tow band can be spread by any of known or common spreading techniques such as a mechanical spreading technique typically with rollers; and a gas spreading technique typically using air. The spreading (blooming) refers to a process (procedure) in which crimped fibers are extended or spread out in a lengthwise direction and/or in a width direction to loosen the fibers so as to increase the bulk of the fibers.

A way to shape the spread fibers into a sheet is not limited and may be selected from known or common methods such as a method of placing the spread fibers between two plate-like articles and pressing the fibers into a sheet; and a method of allowing the spread fibers to pass through between a pair of rollers to be shaped into a sheet.

A non-limiting example of such a method of spreading crimped fibers using a pressurized gas to give spread fibers, and shaping the spread fibers into a sheet is a method in which crimped fibers are fed to a spreading chamber having an elliptic or circular cross section, are spread using a pressurized gas to give spread fibers, and the spread fibers are shaped into a sheet (see JP-A No. 2008-255529). The method as mentioned above can give a fiber sheet that has extremely excellent absorptivity of a liquid such as water. In addition, the method enables easy control of the thickness and width of the fiber sheet, enables partial increase in thickness of the fiber sheet, and can allow the resulting fiber sheet to have a distribution in thickness (to have different thicknesses) in particular in the width direction.

The fiber sheet according to the present invention may have a bulk specific gravity of generally 0.005 to 0.1 g/cm³, and preferably 0.01 to 0.05 g/cm³, although the bulk specific gravity may be set as appropriate according to the intended use and purpose. The fiber sheet according to the present invention may be cut to an appropriate size before use, as appropriate according to the intended use and purpose.

### Absorbent

The absorbent according to the present invention includes the fiber sheet according to the present invention and a superabsorbent polymer (SAP). The superabsorbent polymer is dispersed and disposed within and over the fiber sheet. The superabsorbent polymer (SAP) may be selected from known ones.

The absorbent may be produced typically by dispersing the superabsorbent polymer uniformly in the whole or a predetermined region of the fiber sheet, and hot pressing the resulting article. The superabsorbent polymer may be used in an amount of typically 50 to 1000 g/m², and preferably 100 to 500 g/m² in terms of mass per unit area (METSUKE), although the amount may be set as appropriate according to the intended use and purpose.

The absorbent according to the present invention uses the hydrophilized cellulose acetate tow as an absorbing material, absorbs a liquid (water) in a shorter time, and can absorb, for example, voided urine in a shorter time, as compared with absorbents using fluff pulp. The absorbent has excellent liquid diffusibility, utilizes the superabsorbent polymer (SAP) in a wide area, less causes rewetting typically of urine, and is comfortable to use. In addition, the absorbent has excellent liquid retentivity and restrains spillage of the liquid sideways due typically to baby's motion. The absorbent according to the present invention is therefore useful, in particular, as absorbents for hygiene supplies such as disposable diapers, sanitary napkins (sanitary pads), and breast pads.

### Examples

The present invention will be illustrated in further detail with reference to several examples below. It should be noted, however, that the examples are by no means intended to limit the scope of the present invention.

### Preparation Example 1

A textile oil (mineral oil, trade name DC-18, supplied by Matsumoto Yushi-Seiyaku Co., Ltd.) was tempered to 35°C, was combined with (diluted with) water (dilution water) tempered at 35°C, stirred, and yielded a textile oil emulsion having an emulsion concentration of 40 weight percent.

The textile oil has a phase inversion point of 50 weight percent. The dilution was performed at a dilution rate of 0.13 kg/min per kilogram of the textile oil at the phase inversion point. The emulsion had an average particle size of 0.199 µm. The prepared textile oil emulsion had a total luminous transmittance of 34.8% with respect to light at 850 nm, as measured at an emulsion concentration of 5 weight percent.

### Preparation Example 2

A hydrophilizing component (polyoxyethylene sorbitan monolaurate, having an HLB value of 16.7, supplied by Matsumoto Yushi-Seiyaku Co., Ltd.) was dissolved in water and yielded a hydrophilizing agent aqueous solution having a concentration of 8.5 weight percent.

### Preparation Example 3

A textile oil-hydrophilizing agent mixture was prepared by blending and mixing 30 parts by weight of the textile oil emulsion prepared in Preparation Example 1 with 100 parts by weight of the hydrophilizing agent aqueous solution prepared in Preparation Example 2.

### Example 1

A hydrophilized cellulose acetate tow band was produced using the equipment illustrated in Fig. 1.

A cellulose acetate having a degree of substitution of 2.5 (L-50, supplied by Daicel Corporation) was dissolved in acetone, discharged from spinnerets of spinning cabinets, from which the acetone solvent was volatilized, and yielded cellulose acetate filaments. A multiplicity of the cellulose acetate filaments was bundled in a ropelike manner and yielded cellulose acetate filament tows.

Next, the resulting cellulose acetate filament tows were treated with the textile oil emulsion from Preparation Example 1, using a roller impregnating device to impart (impregnate) the textile oil onto the fiber surface of the cellulose acetate tow. The textile oil was impregnated in an amount of 0.5 weight percent relative to the cellulose acetate tow.

Next, the filaments (cellulose acetate filament tows) from the spinning cabinets (multiple spinning cabinets) were guided via godet rollers, bundled, and yielded a cellulose acetate tow band (tow band). The tow band had a total denier of 24000 and a filament denier of 3.3, where the filaments each had a Y-shaped cross section.

The tow band was treated with the hydrophilizing agent aqueous solution from Preparation Example 2, using a roller impregnating device to impart (impregnate) the hydrophilizing component onto the fiber surface of the cellulose acetate tow. The hydrophilizing component was impregnated in an amount of 0.7 weight percent relative to the cellulose acetate tow.

The tow band imparted with the hydrophilizing component was then subjected to crimping (crimper treatment) in a number of crimps of 36 per inch, dried, packed, and yielded a bale of the hydrophilized cellulose acetate tow band.

The resulting hydrophilized cellulose acetate tow band was spread by the technique (gas spreading technique) described in JP-A No. 2008-255529 and yielded a fiber sheet having a thickness of 1 cm and being derived from (formed from) the hydrophilized cellulose acetate tow band. The spreading was performed under such conditions that the cellulose acetate tow after spreading had a mass of 2 g per an area of 10 cm wide by 30 cm long.

A superabsorbent polymer (collected from trade name Moony Pants Size L, Unicharm Corporation) was dispersed within and over the above-prepared fiber sheet in a mass per unit area (METSUKE) of 250 g/m², wrapped in a tissue (facial tissue), and yielded an absorbent.

### Example 2

An absorbent was produced by an operation similar to that in Example 1, except for impregnating the textile oil in an amount of 0.4 weight percent relative to the cellulose acetate tow, and impregnating the hydrophilizing component in an amount of 0.3 weight percent relative to the cellulose acetate tow.

### Example 3

A hydrophilized cellulose acetate tow band was produced using the equipment illustrated in Fig. 1.

A cellulose acetate having a degree of substitution of 2.5 (L-50, supplied by Daicel Corporation) was dissolved in acetone, discharged from spinnerets of spinning cabinets, from which the acetone solvent was volatilized, and yielded cellulose acetate filaments. A multiplicity of the cellulose acetate filaments was bundled in a ropelike manner and yielded cellulose acetate filament tows.

Next, the resulting cellulose acetate filament tows were treated with the textile oil-hydrophilizing agent mixture from Preparation Example 3, using a roller impregnating device to impart (impregnate) the textile oil and the hydrophilizing component onto the fiber surface of the cellulose acetate tow. The textile oil was impregnated in an amount of 0.5 weight percent relative to the cellulose acetate tow. The hydrophilizing component was impregnated in an amount of 0.4 weight percent relative to the cellulose acetate tow.

Next, the filaments (cellulose acetate filament tows) from the spinning cabinets were guided via godet rollers, bundled, and yielded a cellulose acetate tow band (tow band). The tow band had a total denier of 24000 and a filament denier of 3.3, where the filaments each had a Y-shaped cross section.

The tow band was then subjected to crimping (crimper treatment) in a number of crimps of 36 per inch, dried, packed, and yielded a bale of the hydrophilized cellulose acetate tow band.

The prepared hydrophilized cellulose acetate tow band was spread by the technique (gas spreading technique) described in JP-A No. 2008-255529 and yielded a fiber sheet having a thickness of 1 cm and being formed from the hydrophilized cellulose acetate tow band. The spreading was performed under such conditions that the cellulose acetate tow after spreading had a mass of 2 g per an area of 10 cm wide by 30 cm long.

A superabsorbent polymer (collected from trade name Moony Pants Size L, Unicharm Corporation) was dispersed within and over the above-prepared fiber sheet in a mass per unit area (METSUKE) of 250 g/m², wrapped in a tissue, and yielded an absorbent.

### Example 4

A hydrophilized cellulose acetate tow band was produced using the equipment illustrated in Fig. 1.

A cellulose acetate having a degree of substitution of 2.5 (L-50, supplied by Daicel Corporation) was dissolved in acetone, discharged from spinnerets of spinning cabinets, from which the acetone solvent was volatilized, and yielded cellulose acetate filaments. A multiplicity of the cellulose acetate filaments was bundled in a ropelike manner and yielded cellulose acetate filament tows.

Next, the resulting cellulose acetate filament tows were treated with the textile oil-hydrophilizing agent mixture from Preparation Example 3, using a roller impregnating device to impart (impregnate) the textile oil and the hydrophilizing component onto the fiber surface of the cellulose acetate tow. The textile oil was impregnated in an amount of 0.6 weight percent relative to the cellulose acetate tow. The hydrophilizing component was impregnated in this stage in an amount of 0.4 weight percent relative to the cellulose acetate tow.

Next, the filaments (cellulose acetate filament tows) from the spinning cabinets were guided via godet rollers, bundled, and yielded a cellulose acetate tow band (tow band). The tow band had a total denier of 24000.

The tow band was treated with the hydrophilizing agent aqueous solution from Preparation Example 2, using a roller impregnating device to further impart (impregnate) the hydrophilizing component onto the fiber surface of the cellulose acetate tow in a impregnating amount of 0.4 weight percent relative to the cellulose acetate tow. The hydrophilizing component was impregnated in a total impregnating amount of 0.8 weight percent relative to the cellulose acetate tow.

The tow band imparted with the hydrophilizing component was subjected to crimping (crimper treatment) in a number of crimps of 36 per inch, dried, packed, and yielded a bale of the hydrophilized cellulose acetate tow band.

The resulting hydrophilized cellulose acetate tow band was spread by the technique (gas spreading technique) described in JP-A No. 2008-255529 and yielded a fiber sheet having a thickness of 1 cm and being formed from the hydrophilized cellulose acetate tow band. The spreading was performed under such conditions that the cellulose acetate tow after spreading had a mass of 2 g per an area of 10 cm wide by 30 cm long.

A superabsorbent polymer (collected from trade name Moony Pants Size L, Unicharm Corporation) was dispersed within and over the above-prepared fiber sheet in a mass per unit area (METSUKE) of 250 g/m², wrapped in a tissue, and yielded an absorbent.

### Comparative Example 1

An absorbent was produced by an operation similar to that in Example 1, except for impregnating the textile oil in an amount of 0.9 weight percent relative to the cellulose acetate tow, and not performing the treatment with the hydrophilizing component.

### Comparative Example 2

An absorbent was produced by an operation similar to that in Example 1, except for impregnating, as a hydrophilizing component instead of the one used in Example 1, glycerol (HLB value: 11.1) in an amount of 0.4 weight percent relative to the cellulose acetate tow. The textile oil was impregnated in an amount of 0.5 weight percent relative to the cellulose acetate tow, as in Example 1.

### Comparative Example 3

An absorbent was produced by an operation similar to that in Example 1, except for impregnating, as a hydrophilizing component instead of the one used in Example 1, sorbitan monolaurate (HLB value: 8.6) in an amount of 0.5 weight percent relative to the cellulose acetate tow. The textile oil was impregnated in an amount of 0.5 weight percent relative to the cellulose acetate tow, as in Example 1.

### Comparative Example 4

An absorbent was produced by an operation similar to that in Example 1, except for impregnating, as a hydrophilizing component instead of the one used in Example 1, a polyoxyethylene lanolin (HLB value: 13.0) in an amount of 0.5 weight percent relative to the cellulose acetate tow. The textile oil was impregnated in an amount of 0.5 weight percent relative to the cellulose acetate tow, as in Example 1.

### Comparative Example 5

A cellulose acetate tow band (tow band) was prepared by a procedure similar to that in Example 1. This was impregnated with the textile oil in an amount of 0.5 weight percent relative to the cellulose acetate tow. The tow band had a total denier of 24000 and a filament denier of 3.3, where the filaments each had a Y-shaped cross section.

The tow band was subjected to crimping (crimper treatment) in a number of crimps of 36 per inch and dried. The resulting tow band was introduced into a set of rollers, allowed to pass through a drafting bath at 60°C at a draw ratio of about 1.2 to 1 by the method described in Fig. 1 of Japanese Unexamined Patent Application Publication (Translation of PCT Application) (JP-A) No. H07-504233 to release crimping (to decrimp). A tow band was produced from non-crimped (decrimped) fibers in the above manner. The resulting tow band was treated with the hydrophilizing agent aqueous solution from Preparation Example 2, using a roller impregnating device to impart (impregnate) the hydrophilizing component onto the fiber surface of the cellulose acetate tow. The hydrophilizing component was impregnated in an amount of 0.7 weight percent relative to the cellulose acetate tow.

The tow band imparted with the hydrophilizing component was then cut to a length of 2 inches and yielded short-fiber hydrophilized cellulose acetate filaments.

The short-fiber hydrophilized cellulose acetate filaments were stacked in an amount of 7.5 g per an area of 10 cm wide by 30 cm long and yielded a fiber sheet having a thickness of 1 cm and including the hydrophilized cellulose acetate filaments.

A superabsorbent polymer (collected from trade name Moony Pants Size L, Unicharm Corporation) was dispersed within and over the above-prepared fiber sheet in a mass per unit area (METSUKE) of 250 g/m², wrapped in a tissue, and yielded an absorbent.

### Comparative Example 6

A cellulose acetate tow band (tow band) was prepared by a procedure similar to that in Example 1. In the tow band, the textile oil was impregnated in an amount of 0.5 weight percent relative to the cellulose acetate tow. The tow band had a total denier of 24000 and a filament denier of 3.3, where the filaments each had a Y-shaped cross section.

The tow band was treated with the hydrophilizing agent aqueous solution from Preparation Example 2, using a roller impregnating device to impart (impregnate) the hydrophilizing component onto the fiber surface of the cellulose acetate tow. The hydrophilizing component was impregnated in an amount of 0.7 weight percent relative to the cellulose acetate tow.

The tow band imparted with the hydrophilizing component was then subjected to crimping (crimper treatment) in a number of crimps of 36 per inch, dried, cut into a length of 2 inches with applying sufficient tension, and yielded short-fiber hydrophilized cellulose acetate filaments. The short fibers (filaments) had a number of crimps of 14 per inch.

The resulting short-fiber hydrophilized cellulose acetate filaments were stacked under such conditions to give a mass of 7.5 g per area of 10 cm wide by 30 cm long, and yielded a fiber sheet having a thickness of 1 cm and including hydrophilized cellulose acetate filaments.

A superabsorbent polymer (collected from trade name Moony Pants Size L, Unicharm Corporation) was dispersed within and over the above-prepared fiber sheet in a mass per unit area (METSUKE) of 250 g/m², wrapped in a tissue, and yielded an absorbent.

### Referential Example

A superabsorbent polymer (collected from trade name Moony Pants Size L, Unicharm Corporation) was dispersed in a mass per unit area (METSUKE) of 250 g/m² over the surface of a fiber sheet including pulp short fibers (kraft pulp). The resulting article was wrapped with a tissue and yielded an absorbent. The fiber sheet had a bulk specific gravity of 0.025 g/cm³ and a thickness of 1 cm.

### Evaluation Tests

### Measurement of Impregnating Amounts of Hydrophilizing Component and Textile Oil

About 6 g of each of the cellulose acetate tow bands after imparting of the textile oil and the hydrophilizing component (the tow bands after crimping; for Comparative Example 5, the tow band after decrimping), dried at 105°C for one hour, naturally cooled, and precisely weighed. This was placed into a Soxhlet extractor (supplied by Asahi Glassplant Inc.) to extract the textile oil and the hydrophilizing component. The extraction was performed by refluxing at 100°C for 2 hours using 150 ml of a 7:3 (weight ratio) solvent mixture of hexane and ethyl acetate as an extraction solvent. The solvent was removed after extraction, and the resulting extract was further dried at 105°C for one hour and weighed. The extract weight was divided by the weight of the cellulose acetate tow subjected to the extraction to determine the total impregnating amount (weight percent; relative to the cellulose acetate tow) of the hydrophilizing component and the textile oil.

The extract was then dissolved in deuterated dimethyl sulfoxide (DMSO-d6) and subjected to ¹H-NMR analysis. On the basis of signal intensities in the ¹H-NMR spectrum, the impregnating amounts (weight percent; relative to the cellulose acetate tow) of the textile oil and the hydrophilizing component were determined by the calibration curve method.

### Absorption Performance Evaluation

The absorbents prepared in the examples, comparative examples, and referential example were cut to pieces of a size of 10 cm by 30 cm. The pieces each included 2 g of the tow and 7.5 g of the superabsorbent polymer; but included 7.5 g of the tow and 7.5 g of the superabsorbent polymer for Comparative Examples 5 and 6. The pieces were heated and compressed by hot pressing at 100°C and 3.5 MPa for one minute to adjust the thickness to 2 mm, and yielded evaluation samples.

A cylinder having an inner diameter of 6 cm was placed at the center of each evaluation sample so that the lengthwise direction of the cylinder was orthogonal to the surface of the evaluation sample, and 80 cc of a physiological saline solution were poured into the cylinder. Simultaneously with the pouring, an absorption rate measurement with a stopwatch was started, and the time at which the physiological saline solution disappeared from the evaluation sample surface was defined as a water absorption time (80-cc water absorption) (in second). A sample having a shorter absorption time is considered to be superior in absorption rate.

Further, two portions (second and third portions) of 80 cc of the physiological saline solution were poured to be absorbed by the absorbent 10 minutes and 20 minutes after the pouring of the first portion of the physiological saline solution. After the absorption, the length of an area having a maximum dimension in the longitudinal direction in a region where the physiological saline solution diffused at the absorbent surface was measured with a ruler placed in parallel with the absorbent surface, and the length was defined as a planar diffusion length (240-cc water absorption) (in millimeter (mm)). A sample having a longer planar diffusion length is considered to have superior liquid diffusibility.

### Evaluation of Water Retentivity upon Inclination

The absorbents prepared in the examples, comparative examples, and referential example were cut into a size of 10 cm by 30 cm and yielded evaluation samples.

Each evaluation sample was secured onto an inclined plate secured at an inclination angle of 45 degrees so that the sides of 10 cm be upper and lower sides, and the sides of 30 cm be right and left sides. Toward the upper end portion of the evaluation sample, 80 cc of a physiological saline solution (0.9 weight percent) were dropped at a flow rate of 80 cc/min from a height of about 10 mm above the upper side portion of the evaluation sample. The weight of the physiological saline solution that was not absorbed by the evaluation sample, but leaked out from the lower end of the evaluation sample was measured, and the weight was defined as a liquid retentivity upon inclination (80-cc water absorption) (in cubic centimeter (cc)). A sample having a smaller amount of leaked water (physiological saline solution) in the test for liquid retentivity upon inclination is considered to have superior liquid retentivity.

Results of the evaluations are presented in Tables 1 and 2. In Tables 1 and 2, the term "total impregnating amount" refers to the total of the hydrophilizing component impregnating amount and the textile oil impregnating amount.

### [Table 1]

**TABLE 1**

| | Example 1 | Example 2 | Example 3 | Example 4 |
|---|---|---|---|---|
| Used fibers | Cellulose acetate tow | Cellulose acetate tow | Cellulose acetate tow | Cellulose acetate tow |
| Hydrophilizing component | Polyoxyethylene sorbitan monolaurate | Polyoxyethylene sorbitan monolaurate | Polyoxyethylene sorbitan monolaurate | Polyoxyethylene sorbitan monolaurate |
| HLB value of hydrophilizing component | 16.7 | 16.7 | 16.7 | 16.7 |
| Hydrophilizing component impregnating amount (weight percent) | 0.7 | 0.3 | 0.4 | 0.8 |
| Textile oil | DC-18 | DC-18 | DC-18 | DC-18 |
| Textile oil impregnating amount (weight percent) | 0.5 | 0.4 | 0.5 | 0.6 |
| Total impregnating amount (weight percent) | 1.2 | 0.7 | 0.9 | 1.4 |
| Production method | Method I | Method I | Method II | Method III |
| Water absorption time (sec) | 20.9 | 30 | 27.4 | 21.1 |
| Planar diffusion length (mm) | 235 | 225 | 230 | 235 |
| Water retentivity upon inclination (cc) | 1.9 | 0.8 | 1.8 | 2.2 |

### [Table 2]

**TABLE 2**

| | Comparative Example 1 | Comparative Example 2 | Comparative Example 3 | Comparative Example 4 | Comparative Example 5 | Comparative Example 6 | Referential Example |
|---|---|---|---|---|---|---|---|
| Used fibers | Cellulose acetate tow | Cellulose acetate tow | Cellulose acetate tow | Cellulose acetate tow | Cellulose acetate tow (decrimped) | Cellulose acetate tow (short fibers) | Pulp short fibers |
| Hydrophilizing component | - | Glycerol | Sorbitan monolaurate | Polyoxyethylene lanolin | Polyoxyethylene sorbitan monolaurate | Polyoxyethylene sorbitan monolaurate | - |
| HLB value of hydrophilizing component | - | 11.1 | 8.6 | 13 | 16.7 | 16.7 | - |
| Hydrophilizing component impregnating amount (weight percent) | - | 0.4 | 0.5 | 0.5 | 0.7 | 0.7 | - |
| Textile oil | DC-18 | DC-18 | DC-18 | DC-18 | DC-18 | DC-18 | - |
| Textile oil impregnating amount (weight percent) | 0.9 | 0.5 | 0.5 | 0.5 | 0.5 | 0.5 | - |
| Total impregnating amount (weight percent) | 0.9 | 0.9 | 1 | 1 | 1.2 | 1.2 | - |
| Water absorption time (sec) | 23.8 | 27.3 | 26.2 | 27 | 27 | 25 | 32 |
| Planar diffusion length (mm) | 194 | 230 | 230 | 220 | 230 | 230 | 240 |
| Water retentivity upon inclination (cc) | 24.1 | 8.3 | 10.2 | 5.1 | 3.4 | 2.8 | 1.4 |

### Industrial Applicability

The hydrophilized cellulose acetate tow band according to the present invention maintains liquid permeability, which is a feature of a cellulose acetate tow, and still offers liquid absorbing properties, in particular, liquid diffusibility and liquid retentivity (wetting properties) at higher levels in good balance. The hydrophilized cellulose acetate tow band, as using the cellulose acetate tow, is not bulky and can be reduced in thickness. The hydrophilized cellulose acetate tow band is therefore usable as absorbing materials for absorbents for use in hygiene supplies such as diapers and feminine sanitary products.

### Reference Signs List

- 1: spinneret
- 2: cellulose acetate filament tow
- 3: textile-oil-imparting device
- 4: godet roller
- 5: guide
- 6: tow-band-forming device
- 7: cellulose acetate tow band
- 8: hydrophilizing-component-imparting device
- 9: crimper

## Claims

1. A hydrophilized cellulose acetate tow band comprising:
a crimped cellulose acetate tow band; and
a hydrophilizing component having an HLB value of 16 or more,
the cellulose acetate tow band comprising
a cellulose acetate having a degree of substitution of 2.0 to 2.6,
the cellulose acetate tow band having a total denier of 10000 to 40000 and being crimped in a number of crimps of 30 to 60 per inch,
the hydrophilizing component being impregnated on the cellulose acetate tow band in an amount of 0.3 to 2 weight percent relative to the cellulose acetate tow band.

2. The hydrophilized cellulose acetate tow band according to claim 1,
wherein the hydrophilizing component having an HLB value of 16 or more comprises at least one selected from the group consisting of:
polyoxyethylene sorbitan monolaurates; and
polyethylene glycol monostearates.

3. The hydrophilized cellulose acetate tow band according to one of claims 1 and 2, further comprising
a textile oil,
the textile oil being impregnated on the crimped cellulose acetate tow band in an amount of 0.2 to 2 weight percent relative to the cellulose acetate tow band, in addition to the hydrophilizing component having an HLB value of 16 or more.

4. The hydrophilized cellulose acetate tow band according to claim 3,
wherein the hydrophilizing component having an HLB value of 16 or more and the textile oil are impregnated in a total amount of 0.4 to 3 weight percent relative to the cellulose acetate tow band.

5. The hydrophilized cellulose acetate tow band according to any one of claims 1 to 3,
wherein the crimped cellulose acetate tow band has a filament denier of 1.0 to 7.0.

6. A fiber sheet derived from the hydrophilized cellulose acetate tow band according to any one of claims 1 to 5.

7. A method for producing a hydrophilized cellulose acetate tow band, the method comprising the steps of:
A) dissolving a cellulose acetate having a degree of substitution of 2.0 to 2.6 in an organic solvent to give a spinning dope, and discharging the spinning dope through spinnerets of spinning cabinets to give cellulose acetate filaments;
B) coating the cellulose acetate filaments with a textile oil;
C) coating the cellulose acetate filaments with a hydrophilizing component;
D) unifying the cellulose acetate filaments from the spinning cabinets to give a cellulose acetate tow band having a total denier of 10000 to 40000; and
E) crimping the cellulose acetate tow band to give a crimped cellulose acetate tow band having a number of crimps of 30 to 60 per inch.

8. The method according to claim 7 for producing a hydrophilized cellulose acetate tow band,
wherein the step B) comprises
coating the cellulose acetate filaments with the hydrophilizing component in addition to the textile oil; and
wherein the step C) comprises
further coating the cellulose acetate filaments with the hydrophilizing component, where the cellulose acetate filaments having been coated with the textile oil and the hydrophilizing component.

9. The method according to one of claims 7 and 8,
wherein the steps B), D), and C) are performed in this sequence.
